# EUROPEAN PATENT SPECIFICATION

(11) **EP 3 986 339 B1**
(45) Date of publication and mention of the grant of the patent: **16.09.2026**
(21) Application number: 20827308.6
(22) Date of filing: 17.06.2020
(51) Int. Cl.: A61F 2/80, A61F 2/50, A61F 2/54, A61F 2/60, A61F 2/64, A61F 2/76, A61F 2/78

(54) **AIR HAMMOCK FOR A PROSTHETIC**
LUFTHÄNGEMATTE FÜR EINE PROTHESE
HAMAC PNEUMATIQUE POUR PROTHÈSE

(30) Priority: 18.06.2019 US 201962863122 P; 16.06.2020 US 202016902712
(43) Date of publication of application: 27.04.2022
(73) Proprietor: Martin, James Jay, Oklahoma City OK 73101 (US)
(72) Inventor: Martin, James Jay, Oklahoma City OK 73101 (US)
(74) Representative: Cleveland Scott York
(86) International application number: PCT/US2020/038059
(87) International publication number: WO 2020/257233

(56) References cited:
- EP-A1- 2 775 967
- US-A1- 2009 082 877
- US-A1- 2012 101 597
- US-A1- 2017 143 518
- US-A1- 2018 153 716
- US-A1- 2018 153 716
- US-A1- 2018 235 785
- US-A1- 2018 296 373
- US-A1- 2019 110 910

## Description

### BACKGROUND OF THE INVENTION

### 1. FIELD OF THE INVENTION

The present invention relates generally to a limb prosthesis or orthosis for amputees or orthotic users. More particularly, the present invention is a new and improved socket that provides dynamic conformity to better match to the underlying limb shape in real-time through a combination of structural support members with modularly adjustable compliant members thereby creating an air hammock for a prosthetic by suspending the limb within the socket. It is further contemplated to provide a computer controlled non-back drivable gear mechanism to adjust the circumference of the prosthetic interface to match as desired the circumference of the residual limb.

### 2. BACKGROUND OF THE INVENTION

It is estimated that by the year 2050, the number of amputees will double to over 3.6 Million. American population health concerns are strongly correlated to the weight and age of the individual. The American population and its health patterns show an increase in all high-risk areas as related to dysvascular disease, a leading cause of both stroke and amputation. In the United States, 97% of dysvascular related amputations involve the lower limb. The aging baby-boomer population is just entering the age where vascular insufficiencies tend to drastically increase, thus, a large influx of stroke patients and amputee patients will be entering the U.S. market alone in the coming few years.

Likewise, there exists over 30,000,000 amputees in developing nations, where less than 5% will have access to well-fitting prosthetics in their lifetime. Three main causes of such a dramatically underserved amputee population in developing nations are conventional prosthetics are relatively expensive to fabricate and fit; there is a lack of expensive equipment needed to fabricate conventional prosthetics; and there is a lack of trained and skilled practitioners who are able to fit them.

The prosthetics market has advanced significantly in recent years now utilizing many new advanced components such as computer controlled knees and feet. However, the socket interfaces are minimally different from what was being used 20 to 30 years ago. In fact, many of the socket interfaces that are still considered state-of-the-art were originally developed between the 1960's and 1990's, with only minor advancements in materials and suspension methods since then. The core socket interface designs have gone largely unchanged.

Conventionally used prosthetic interfaces remain as an anatomically contoured socket in which the residual limb fits within. This socket may be specifically tailored to the residual limb's size and shape, but it largely remains as a static size and shape.

While some flexible materials may be incorporated, their flexibility is typically no more than minor amounts of give at their edges, and not true accommodation for the dynamic nature of the underlying body in which they are fit. There are numerous prosthetic and orthotic companies that provide components for conventional interface designs ranging from various embodiments of gel liners and suspension aids for prosthetics users. In each of these, the core interface approaches used are antiquated and in desperate need of more advanced methods of how to fit prosthetic devices.

In the field of clinical prosthetics, the conventional prosthetic sockets have historically used static rigid materials that are anatomically contoured to match the underlying limb shape typically using carbon fiber or other laminates, thermoplastic shells of various stiffnesses each of which has a shape, contouring, and size, which is largely or wholly set. Each of the prior art embodiments of prosthetic sockets utilizes largely un-conforming elements that surround and encapsulate the underlying limb to create a structure to support the user's limb within the socket. The shape of such structural elements is specifically anatomically contoured to the underlying limb shape in order to provide an anatomical match for the user.

Conventional teachings suggests that the residual limb needs to be encapsulated in rigid or semi-rigid structure to support the user through a hydrostatic or specific pressure loading method as is conventionally used in the field of clinical prosthetics. However, the dynamic limb anatomy changes frequently (daily and/or over a period of time) resulting in the dynamic limb shape no longer matching to the static socket shape. Certain attempts have been made in creating adjusting sockets, although the prior art still utilizes the same encapsulated, rigid, static methods to accomplish the same, though with certain areas of the socket being adjustable.

For instance, the Revo-Fit socket system uses adjustable rigid panels that can press inward or outward, though the overall socket design remains rigid and unconforming. The CJ Sail socket system uses a compliant panel attached to a rigid static socket frame. This design, while more conforming than others, requires a large portion of the limb to rest against a static, rigid socket frame. Additionally, the single compliant member of that design fails to fully conform along the length of the limb, or conform around the circumferential shape of the limb - since the human limb shape changes in contouring with volume gain or loss along both the length and circumference of the limb.

Through using compliant dynamic socket materials that encapsulate the limb, the limb muscles no longer hit a rigid wall, and therefore have the ability to more effectively contract and hypertrophy. In conventional rigid sockets, the limb muscles may hit a rigid socket wall, preventing them from contracting, and hence the limb muscles often atrophy over time. As the limb muscles are now allowed to contract more effectively because of the use of the compliant dynamic socket materials, the muscles therefore exhibit greater blood flow and circulation, and hence greater limb health.

The current state-of-the-art fails to truly accommodate for limb volume changes, has narrow transitions from high force to no force resulting in rubbing and discomfort, and creates a volatile skin environment that is hot and sweaty. In recent years there have been a few attempts at improving lower extremity socket interface design, and overcome some of the outstanding issues surrounding them.

US2018153716A1, which discloses the preamble of claim 1, describes a transfemoral prosthetic socket apparatus for attaching a prosthetic to a limb of a user including first and second stabilizer units, first and second adjustable members, and a floating member positioned between said first said second stabilizer.

US20180235785A1 describes an adjustable socket system that includes a distal portion and proximal portion, wherein an axis extends between the distal and proximal portions, a plurality of struts connected to the distal portion distributed circumferentially about the axis, and a tightening system operatively connected to the struts arranged to differentially tighten and loosen the fit of the adjustable socket system.

EP2775967A1 describes a modular prosthetic system comprising a prosthetic socket for a residual limb, including one or more struts arranged longitudinally; one or more proximal brim members; and one or more distal socket members, wherein one or more of the socket component groups comprise modular components.

It is, therefore, desirable to provide a new and improved socket that provides additional areas of the limb that can be contained and controlled through more dynamic and compliant materials, versus rigid or semi-rigid support members, where the limb can be suspended within the socket with predominantly compliant dynamic materials, which more effectively contour to the underlying limb shape, and results in a more comfortable socket where the prior art is deficient. The above discussed limitations in the prior art are not exhaustive. The current invention provides an inexpensive, time saving, more reliable apparatus where the prior art fails.

### SUMMARY OF THE INVENTION

In view of the foregoing disadvantages inherent in the known types of prosthetic interface design, the present invention provides a new and improved, apparatus for providing control and comfort within a prosthetic device in a more efficient and effective manner. As such, the general purpose of the present invention, which will be described subsequently in greater detail, is to provide a new and improved apparatus according to claim 1.

In a preferred embodiment, the invention may comprise an air hammock for a prosthetic with dynamic conformity to better match to the underlying limb shape in real-time through a combination of structural support members with modularly adjustable compliant members thereby creating an air hammock for a prosthetic by suspending the limb within the socket without pressure on the bottom of the residual limb.

In this respect, before explaining at least one embodiment of the invention in detail, it is to be understood that the invention is not limited in this application to the details of construction and to the arrangement of the components set forth in the following description or illustrated in the drawings. The invention is capable of other embodiments and of being practiced and carried out in various ways. Also, it is to be understood that the phraseology and terminology employed herein are for the purpose of description and should not be regarded as limiting. As such, those skilled in the art will appreciate that the conception, upon which this disclosure is based, may readily be utilized as a basis for the designing of other structures, methods and systems for carrying out the several purposes of the present invention. It is important, therefore that the claims be regarded as including such equivalent constructions insofar as they do not depart from the spirit and scope of the present invention.

Accordingly, titles, headings, chapters name, classifications and overall segmentation of the application in general should not be construed as limiting. Such are provided for overall readability and not necessarily as literally defining text or material associated therewith.

Further, the purpose of the foregoing abstract is to enable the U.S. Patent and Trademark Office and the public generally, and especially the scientist, engineers and practitioners in the art who are not familiar with patent or legal terms or phraseology, to determine quickly from a cursory inspection the nature and essence of the technical disclosure of the application. The abstract is neither intended to define the invention of the application, which is measured by the claims, nor is it intended to be limiting as to the scope of the invention in any way.

It is, therefore, an object of the present invention to provide a new and improved dynamic prosthetic interface for a better match to the underlying limb shape in real-time through a combination of structural support members with modularly adjustable compliant members thereby creating an air hammock for a prosthetic by suspending the limb within the socket.

It is a further object of the present invention to provide a prosthetic interface that may utilize, flexible bands and or cross connectors that may span between structural members and floating or anchored struts to circumferentially surround the limb, and provide support.

Another further object of the present invention is to provide a prosthetic interface with an even greater surface area of the residual limb that may be supported by materials of greater conformity to provide even greater comfort.

Even another further object of the present invention is to provide a prosthetic interface with an even greater surface area of the residual limb that may be supported by materials of greater conformity to provide even greater comfort that is simpler and more consistent to which is user adjustable.

Still another further object of the present invention is to provide a prosthetic interface that matches a user's needs, including through using materials of various stiffnesses, flexibility, stretch, texture, more breathable, and containment.

It is also another further object to provide a computer controlled non-back drivable gear mechanism to adjust the circumference of the prosthetic interface to match as desired the circumference of the residual limb.

It is a further object of the present invention to provide a prosthetic interface that is lower profile under clothing and lighter in weight.

It is a further object of the present invention to provide a prosthetic interface that is modular and repairable.

It is a further object of the present invention to provide a prosthetic interface that can be fabricated less expensively and quicker.

Yet another further object of the present invention is to provide a prosthetic interface wherein additional areas of the limb can be contained and controlled through more dynamic and compliant materials, versus rigid or semi-rigid support members.

It is a further object of the present invention to provide a prosthetic interface that truly accommodates for volume and shape changes of the dynamic underlying body wherein the limb can be suspended within the socket with predominantly compliant dynamic materials, which more effectively contour to the underlying limb shape, and result in a more comfortable socket.

It is still a further object of the present invention to provide a prosthetic interface that does not encapsulate the limb in the same manner as conventional designs.

It is still yet a further object of the present invention to provide a prosthetic interface that captures the lost biomechanical and neuromuscular connection between the limb and the user.

It is also is a further object of the present invention to provide a prosthetic interface to better control underlying bone position within the socket.

Another object of the present invention is to provide a new and improved system, which provides some of the advantages of the prior art, while simultaneously overcoming some of the disadvantages normally associated therewith.

These together with other objects of the invention, along with the various features of novelty that characterize the invention, are pointed out with particularity in the claims annexed to and forming a part of this disclosure. For a better understanding of the invention, its operating advantages and the specific objects attained by its uses, reference would be had to the accompanying drawings and descriptive matter in which there are illustrated preferred embodiments of the invention.

### BRIEF DESCRIPTION OF THE PICTORIAL ILLUSTRATIONS, GRAPHS, DRAWINGS, AND APPENDICES

The invention will be better understood and objects other than those set forth above will become apparent when consideration is given to the following detailed description thereof. Such description makes reference to the annexed pictorial illustrations, graphs, drawings, and appendices.
Figure 1 generally illustrates an embodiment in accordance with the current invention.
Figure 2 generally illustrates an embodiment in accordance with the current invention.
Figure 3 generally illustrates an embodiment in accordance with the current invention.
Figure 4 generally illustrates an embodiment in accordance with the current invention.
Figure 5 generally illustrates an embodiment in accordance with the current invention.
Figure 6 generally illustrates an embodiment in accordance with the current invention.
Figure 7 generally illustrates an embodiment in accordance with the current invention.
Figure 8 generally illustrates an embodiment in accordance with the current invention.
Figure 9 generally illustrates an embodiment in accordance with the current invention.
Figure 10 generally illustrates an embodiment in accordance with the current invention.
Figure 11 generally illustrates an embodiment in accordance with the current invention.
Figure 12 generally illustrates an embodiment in accordance with the current invention.
Figure 13 generally illustrates an embodiment in accordance with the current invention.
Figure 14 generally illustrates an embodiment in accordance with the current invention.
Figure 15 generally illustrates an embodiment in accordance with the current invention.
Figure 16 generally illustrates an embodiment in accordance with the current invention.
Figure 17 generally illustrates an embodiment in accordance with the current invention.
Figure 18 generally illustrates an embodiment in accordance with the current invention.
Figure 19 generally illustrates an embodiment in accordance with the current invention.
Figure 20 generally illustrates an embodiment in accordance with the current invention.
Figure 21 generally illustrates an embodiment in accordance with the current invention.
Figure 22 generally illustrates an embodiment in accordance with the current invention.
Figure 23 generally illustrates an embodiment in accordance with the current invention.
Figure 24 generally illustrates an embodiment in accordance with the current invention.
Figure 25 generally illustrates an embodiment in accordance with the current invention.
Figure 26 generally illustrates an embodiment in accordance with the current invention.

### DETAILED DESCRIPTION OF THE PREFERRED EMBODIMENTS

Referring now to the drawings, wherein like reference numerals designate corresponding structure throughout the views, and referring in particular to the figures, numeral 10 generally refers to a new and improved compliant based transfemoral level prosthetic socket apparatus, assembly, and or system, hereinafter referred to generally and collectively as invention 10.

Of note, invention 10 may be generally shown by example in a configuration for an individual missing a right or left leg or portion thereof at a knee disarticulation or transfemoral level. It is understood that such configuration is for example purposes only and that such should not be considered limiting and a left or right side configuration is also considered. It is further understood that invention 10 may be used where the level of amputation may dictate a different configuration than transfemoral or knee disarticulation level, such as but not limited to transtibial, transradial, transhumeral, or other levels either prosthetically, orthotically, or with exoskeletal robotics, all of which may be considered as human/machine connectivity.

The terms should not be considered limiting the invention nor the general shape and configuration depicted in the drawings. Invention 10 may encompass many embodiments, as generally illustrated in the various figures, and should not be considered limiting where any particular figure depicts one embodiment of invention 10, as there are various elements, embodiments, and user specific requirements. Likewise, the number or placement of anchored or floating struts should not be considered limiting, as any strut may be anchored or floating, and their position for example may use floating struts on the anterior and anchored on the posterior aspect of the socket as well for example.

In a preferred construction, there may be a distal attachment area or structure for mounting other prosthetic components to, such as but not limited to knees, feet, stubbers, connectors, or other conventionally used components which are used distal to a socket apparatus. The particular attachment means may be any conventionally used means, including plates, screws, gunk, and others.

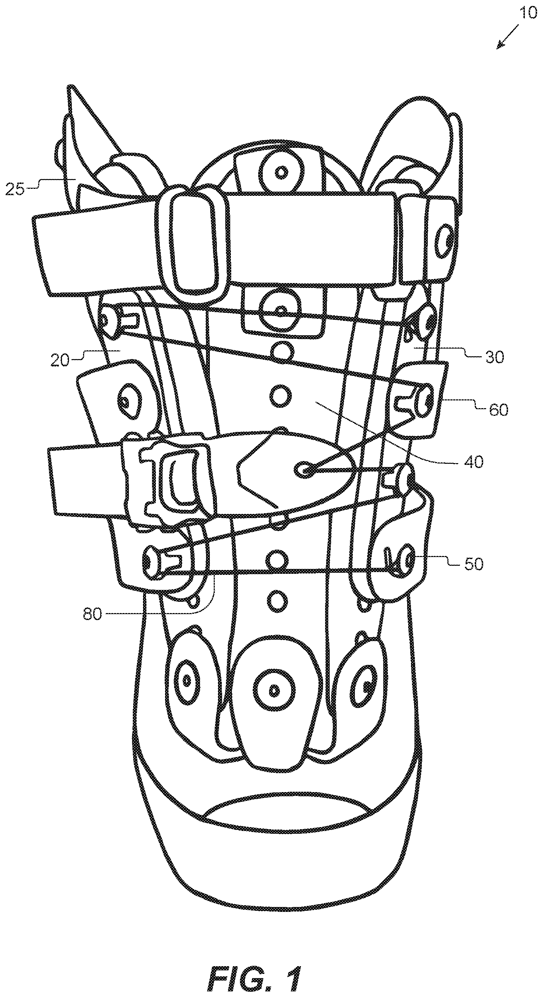

Figure 1 generally illustrates a preferred embodiment for a socket 25 with a first side 20 and a second side 30, which may be two posterior vertical supporting members, and or floating struts, with a middle floating strut 40 in between to encapsulate the limb with modular dynamic adjustable members 50 and 60. This illustration is shown with conformable modular dynamic members 50 and 60 that help circumferentially contain the limb and do not put any pressure on the residual limb distal end. This illustration exhibits the use of a lacing system 70 with a ratchet and ladder strap 80 to tighten the two floating posterior struts together.

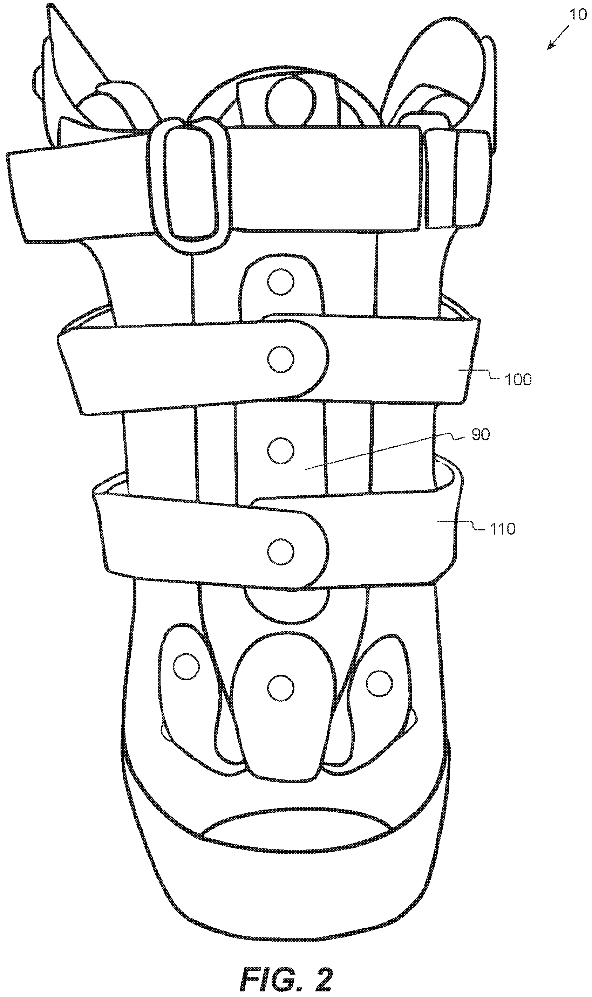

Figure 2 generally illustrates a preferred embodiment using one posterior strut 90, instead of two or three, to encapsulate the limb. This illustration is shown with conformable modular dynamic members 100 and 110 that help circumferentially contain the limb, and can also include adjustability as desired.

In each of the Figures 1 and 2 configurations, the limb is encapsulated with modular dynamic members attached to anchored and/or floating struts to circumferentially support the limb and match to the user's limb shape, and may have adjustable means. The circumferential supporting modular dynamic members may be dynamically flexible enough to contour to the limb shape and generally wrap around the limb shape, while the vertical supporting members may have enough structural integrity to help post and support the limb tissue such that it prevents the circumferential members from roping into the limb tissue.

Through using one, two, three, or more vertical members, or struts, the limb remains supported while the modular dynamic circumferential members flexes to allow for circumferential contouring of the socket about the limb shape. In such illustrations, the distal receptacle and vertical struts are floating, and suspended from the anterior support structure via the circumferential components. It is understood that both the vertical and or the circumferential members may have varying degrees of flexibility, conformity, or rigidity to accomplish the goal of contouring to the limb's shape.

Any such members, including the circumferential members may be fabricated of various types of materials including thermoplastics, textiles, webbing, or other such materials that exhibit such conformability characteristics. It is understood that in this embodiment and others illustrate that the terms webbing and others should not be considering limiting, and may include a variety of materials that may exhibit ultra-conforming characteristics, and may include materials such as nylon webbing, polyester webbing, polypropylene webbing, nomax webbing, kevlar webbing, or a variety of other materials used in similar applications such as lacing, wires, or others.

Likewise, the descriptive term webbing may utilize various embodiments of lacing, including cables, strings, bands, or other materials used in lacing applications, all of which shall be considered one and the same, as each of the descriptive of the materials are conforming, may be placed through guides, routed in various patterns, and or used to tighten an object around the body, similar to clothing or shoe applications.

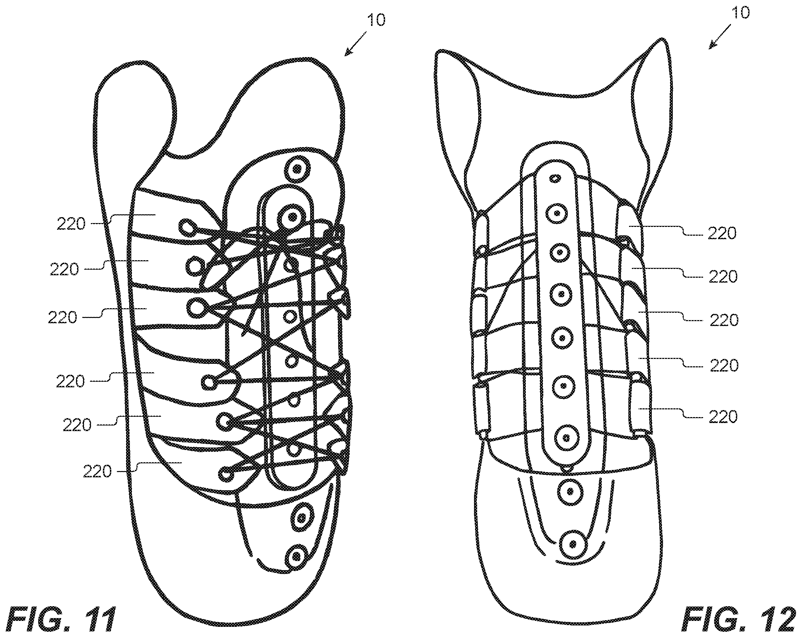

It is contemplated that any such webbings or lacing methods may provide a self-leveling means of accommodating for high force areas within the socket, similar to how a sneaker's laces may self-level in tightness between the various laces, assuming a low-friction lacing system. In such systems, when laces are pulled tight, the 'pulley system' of the laces causes any certain tight lace to loosen via the adjoining laces tightening to equalize forces between laces. It is contemplated that a webbing system may be combined with a lacing system specifically in order to allow for a greater amount of self-leveling in tightness across the limb, as generally illustrated in Figures 11 and 12.

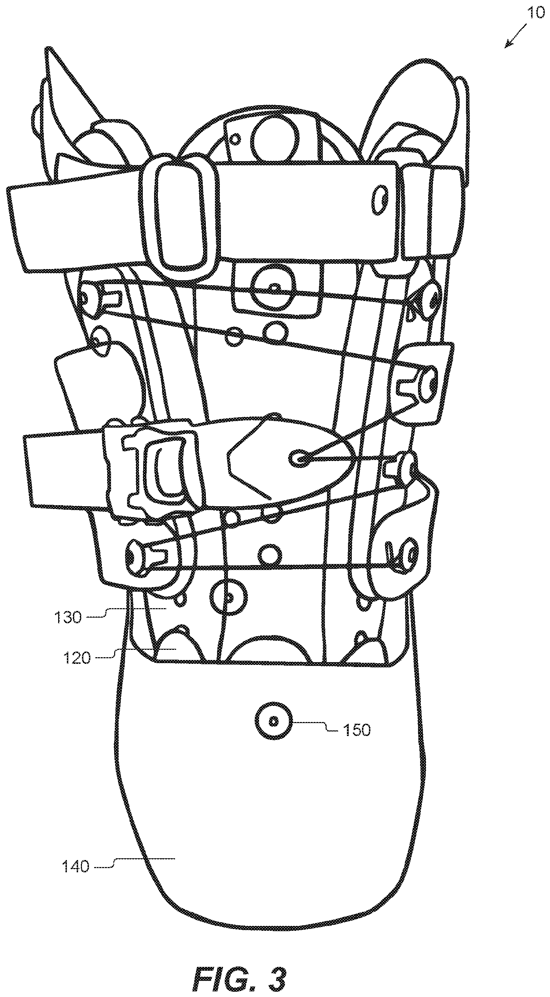

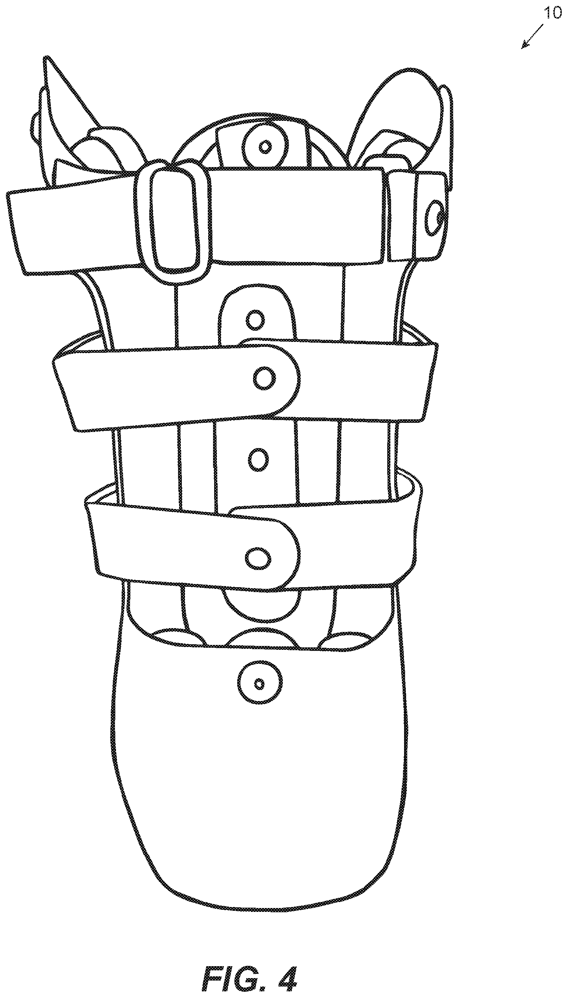

Figure 3 and Figure 4 illustrate similar socket embodiments as Figures 1 and 2 although integrates the distal receptacle 120 and or vertical support member strut assembly 130 into a posterior distal aspect of a frame structure 140 via at least one mounting point 150. In such an embodiment, the distal aspect of the strut may be mounted, though still allow for the proximal end to dynamically adjust in an anterior/posterior, and even possibly medial/lateral aspects, as the connection point may exhibit a certain level of living hinge characteristics, or other similar means to accomplish the same flexibility yet stability. In such an example, the use of a distal receptacle may be integrated to support the distal aspect of the limb, or the limb may rest into or within the distal aspect of the frame mount, which may be contoured to generally match the distal aspect of the limb shape.

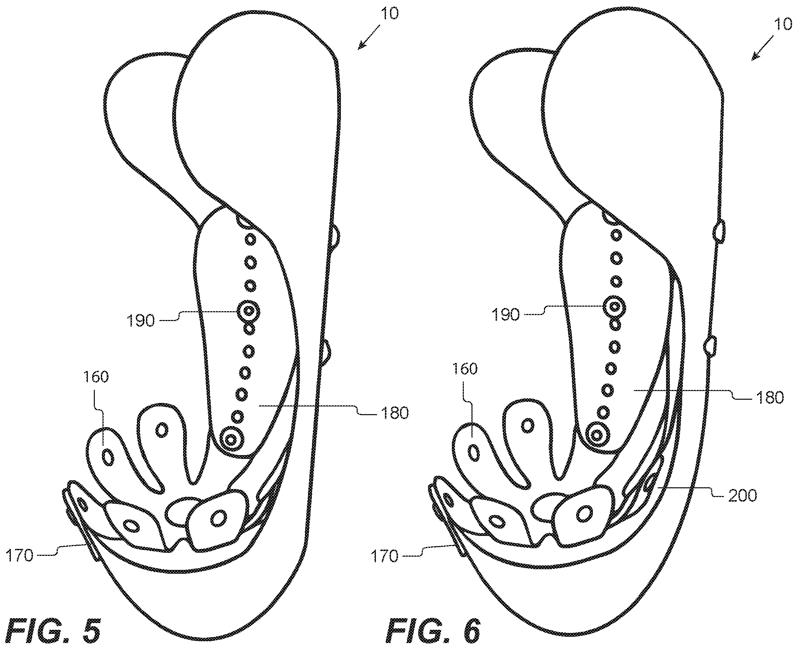

Figures 5 and 6 illustrate a distal receptacle 160, which may be mounted to the posterior vertical support(s) mounting location to a frame 170, as well as the anterior rigid support member directly or via a suspended flexible support member 180, which may be mounted to an anterior rigid support member 190. Flexible vertical members may be suspended from the inside of the anterior support structure member more proximally, and allowing their distal aspect to hang within the structure. In such an example the distal receptacle 160 may be mounted to the anterior flexible support members 180, which may be mounted to the anterior support structure 190, thereby allowing the distal receptacle 160 to be suspended by both the anterior and posterior aspect of the socket configuration, and provide support of the limb but through compliant materials, versus the end of the limb resting into a rigid structural element. In one such embodiment, the posterior aspect of the receptacle may be mounted to the distal aspect of the posterior strut and/or posterior frame section assembly, while the anterior aspect of it may hang from the anterior vertical support members, and therefore allowing it to float within a main support structure 200 like a hammock. In such an example, as weight of the limb is pressed into the distal receptacle, it may promote the receptacle to pull away from the anterior main support structure 200, thereby preventing pressure against a rigid structure during weight bearing.

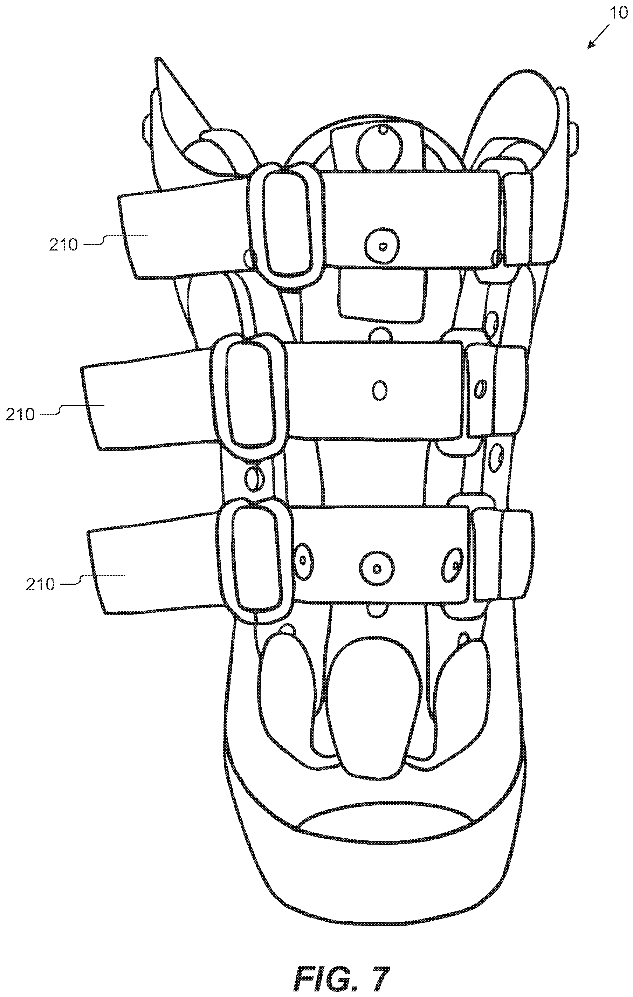

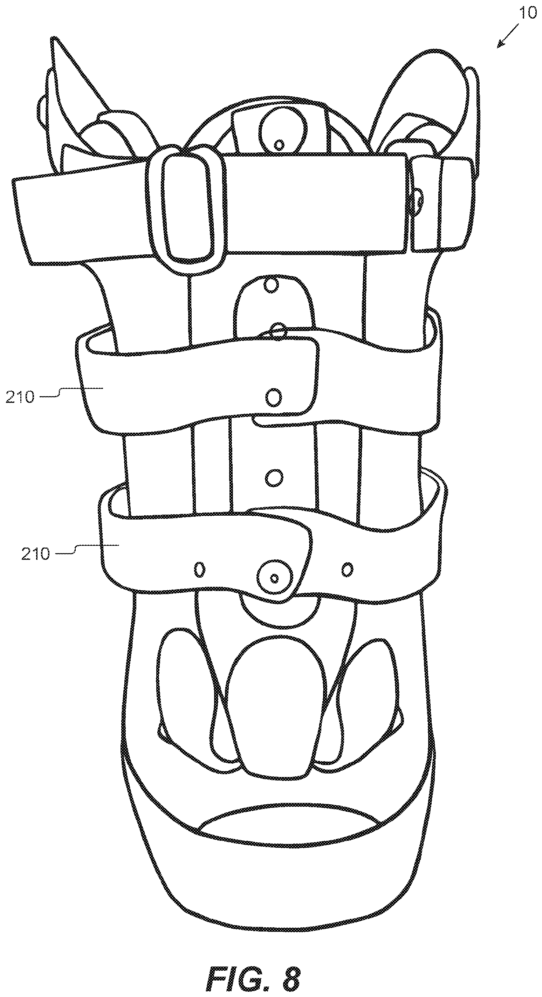

Figures 7 and 8 illustrate similar socket embodiments as Figures 1 and 2 although may integrate a more compliant webbing 210 in place of thermoplastic circumferential connectors. Through using a more compliant material such as webbing, the socket sub-components may more effectively contour to the limb shape. It is anticipated that various materials may be used here, and such description should not be considered limiting. It is contemplated that through using a more compliant material such as a webbing that various lacing configurations may be used to support the limb in various ways. It is understood that the term lacing is generally used in this context to describe the routing, mounting, adjustability, security, and/or positioning of such a webbing or the like. For example, similar to how laces are used in a shoe, various configurations may be used on how the laces are routed through the eyelets, how they are attached, where they are attached, how they are secured, and other such considerations.

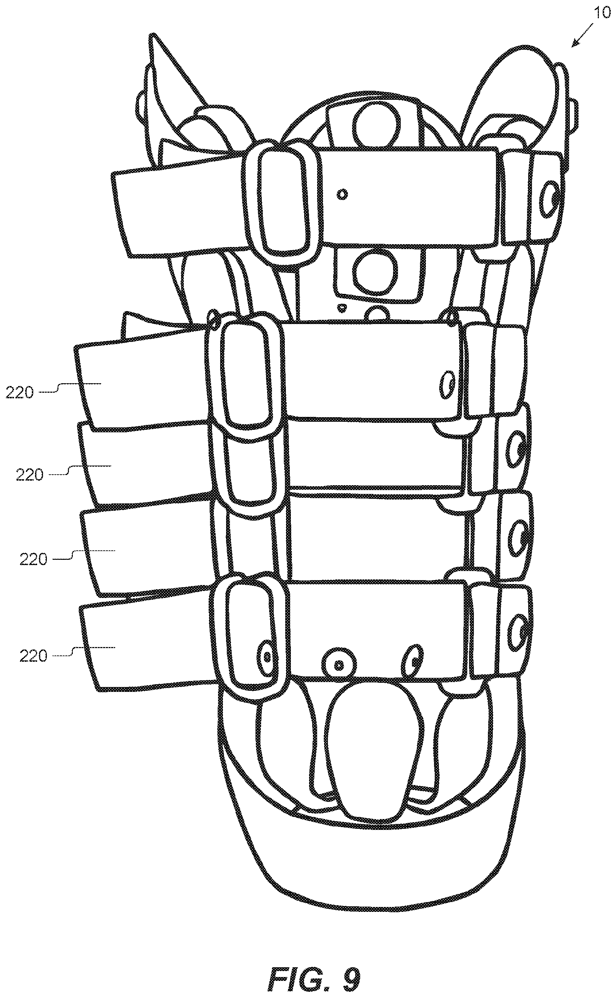

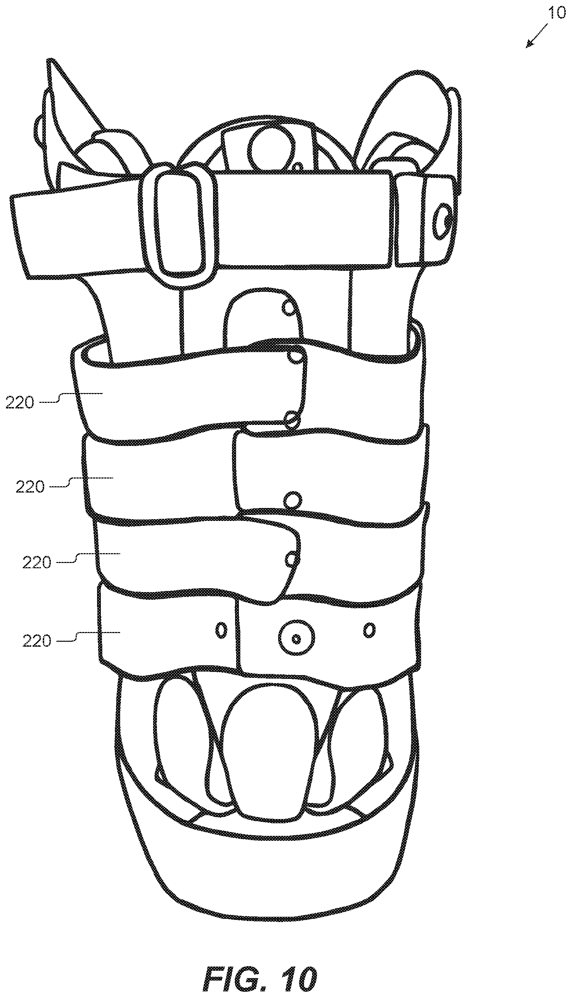

Figures 9, 10, 11, and 12 illustrate various configuration embodiments where various numbers of such webbing material(s) 220 may be wrapped around the limb, such as one, or many. Each of the following cross section views of the socket may as well utilize various numbers of webbings around the limb, including one webbing wrapped around the limb, or multiple webbings wrapped around the limb. In a preferred embodiment, various numbers of webbings wrapped around the limb may be advantageous as it may allow for more specific adjustability to the limb anatomy, discrete individual adjustments to be made at various limb levels, or a unique combination of lacing patterns to more effectively capture and control the limb tissue.

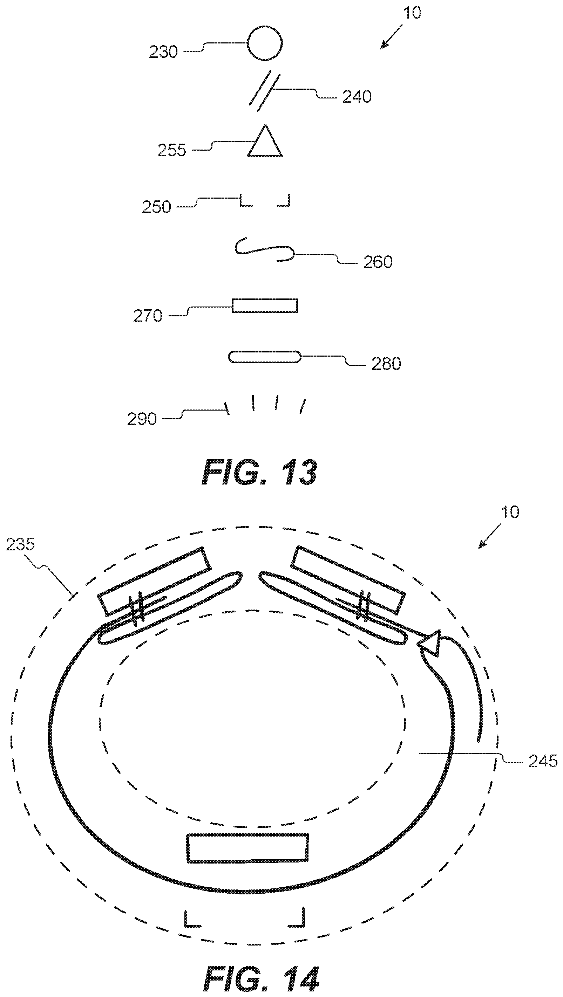

In the various Figures 13 through 20 configurations, the modularity of such webbing may be routed in various patterns, anchored in various methods or positions, and integrate various adjustability or locking mechanisms wherein directional loops 230, attachment and or anchor point 240, buckle and or adjustment mechanism ratchet 255, lace and or web guide 250, webbing 260, anchored or floating strut and or frame 270, suspended flexible member 280, and insert connector 290 are generally depicted in figurative format
The illustrations simply suggest various possible embodiments to accomplish the same. In a preferred embodiment, the proposed anterior struts (proximal on the illustrations) may be anchored supports while the posterior strut (distal on the illustration) may be floating or distally mounted or anchored yet conforming and/or adjustable to the limb. In such examples, and in others, any such strut may be floating or anchored in the various configurations.

Likewise, while two anterior struts are shown, and one posterior strut is shown, it is contemplated that various numbers of struts may be used, including one anterior strut, two posterior struts, or other numbers. Similarly, the term strut should not be considering limiting, and generally refers to an area where webbings or the like may be mounted or supported. The strut may be a modular component, which can be assembled onto the socket in various positions, angles, contouring's, or lengths, and allow the socket assembly to mount thereto, or the strut may be a custom molded form based on the limb anatomy.

The illustration of two anterior struts in a preferred embodiment may enable a simplified design for durability, minimization, and ease of assembly. For comparison, one anterior strut is contemplated though may require additional complexities for durability, rigidity, and assembly. Using two or more struts on the posterior side are contemplated but may add components and complexities compared to a single posterior strut assembly. If one anterior strut were used, and two posterior struts, for example, the webbing may be able to more effectively contour under the tibial condyles to help support the limb, though this is not a preferred embodiment. Regardless of the type of strut, the modular assembly of such a system may be accomplished by capturing a digital or physical impression of the limb and assembling the modular socket components over such impression, or may be built over the limb directly. By way of example, a cast of the limb may be captured using fiberglass wrap, or similar materials, and may include an alienable component within to attach to other prosthetic components. The cast's trim lines may be cut to preference, and holes drilled for mounting the modular socket components thereto. The cast shape may functionally become the anchored struts. The modular socket components may be assembled to match the user's limb shape, and adjustments may be made on the limb. Mounting modularly connected floating or anchored struts may be accomplished by physically connecting a portion of such strut to a portion of the cast frame section, and which may be connected using materials or means which provide a sort of living hinge at the connection point, allowing the connected strut to be controlled in where and how it may move relative to the frame assembly.

Figure 14 illustrates an embodiment where the webbing may be mounted to one anchored strut, wrapped around the limb, through a guide connecting to a possible adjoining floating or anchored strut, and through an adjustable mechanism, which may be attached to webbing connecting to yet another strut. In such an example, a single or multiple webbings may be used to wrap circumferentially around the limb top to bottom for instance, and may or may not include an adjustable mechanism to adjust the length of the webbing. It is contemplated that the webbing may or may not directly interact with or be physically connected to the illustrated middle strut, but in a preferred embodiment, it may be either physical connected or may use a guide to virtually connect it to the strut, thereby allowing for it to hence control the position, compression, or other dynamics of such a strut with respect to the underlying limb. It is contemplated that interaction or connectivity to such a strut enables for the span of the webbing to be controlled around the limb tissue, by the strut posting the limb tissue such that the webbing does not rope into the limb tissue, and provide additional bony or anatomical control of the limb. Residual limb circumference 245 is generally to illustrate the prosthetic interface circumference 235. The depictions are for general purposes and not intended to show actual fit and or circumferences.

It is contemplated that a floating strut may as well not connect to any anchored portion of the system, and may simply be attached to the webbing alone such that it helps post the limb tissue as the webbing wraps around the body. In such an example, there may be one or various webbing members, typically oriented above and below each other as they each wrap around the limb from top to bottom for example. A true floating strut may be attached to each of the various webbing members, for instance vertically, connecting two or more webbing members, and may be a modular element to provide added pressure within a certain area of the socket.

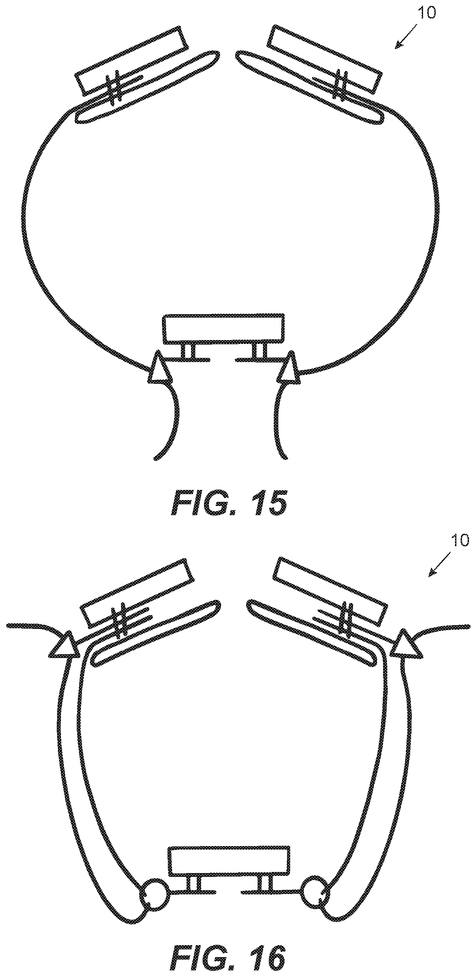

Figure 15 illustrates an embodiment where multiple circumferential webbings may be mounted to anchored struts for instance, around a portion of the limb, and through an adjustor mechanism on an adjoining strut. In any such example, the adjuster mechanism may use any method used to secure the webbing within the system, and prevent it from loosening. This may include, but not limited to, backpack-type buckles, ratchets of various types, Velcro, and others. It is contemplated that a myriad of various types of such adjustor mechanisms may be used to accomplish the same.

Figure 16 illustrates an embodiment where at least one webbing may be mounted to a strut, routed through a direction loop on an adjoining strut, and back through an adjustor on the other strut. In such an example, the added directional loop provides mechanical advantage for pulling the webbing tighter on the limb.

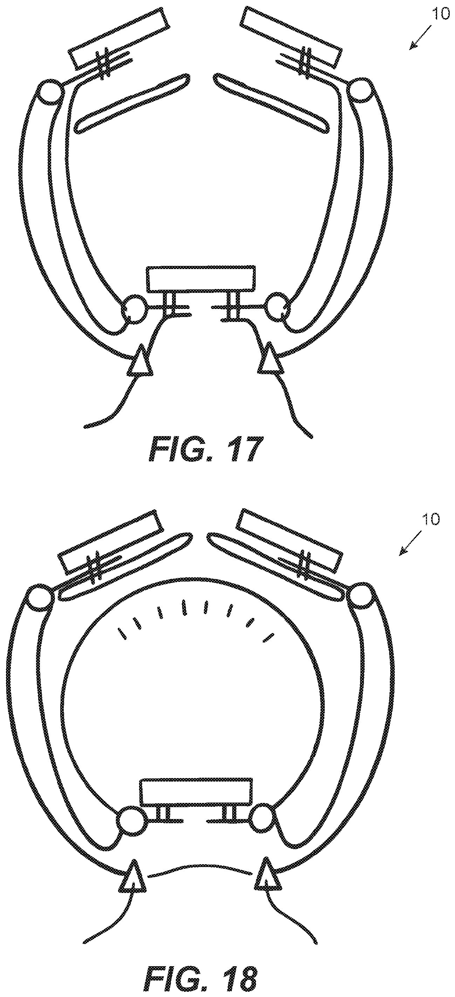

Figure 17 illustrates an embodiment where at least one webbing may be mounted to a strut, routed through a directional loop on an adjoining strut, back through another directional loop, and then through an adjustor, in order to provide additional mechanical advantage. It is contemplated that any number of directional loops may be included to provide additional mechanical advantage.

Figure 18 illustrates an embodiment where the webbing may be floating, and not directly hard-connected to the struts as in other illustrations, and may be routed through at least one directional loop, and then may be routed through an adjustor mechanism. In such an example, by not hard-connecting the webbing loop to the struts, and allowing it to instead float within the socket, as the webbing is tightened through the adjustor mechanism, it may both pull a connected floating strut toward the other anchored struts and simultaneously may pull the limb back toward the connected floating strut. In the example of a below knee prosthetic socket, the posterior strut (distal strut in the illustration) may be connected or mounted at its distal aspect to the frame, which may allow its proximal end to bend forward as the socket is tightened, while maintaining its distal aspect pulled back toward the posterior distal aspect of the frame. As the webbing is tightened, the residual limb may be pulled back posteriorly at its distal aspect via the adjustable webbing, while the proximal end of the limb may be pressed forward toward the anterior anchored struts. This may be biomechanically advantageous, as the sensitive distal aspect of the limb may be supported through a more compliant webbing material versus hitting a rigid structure.

In order to help simplify the integration of various webbings wrapping around the limb, an additional insert may be used, which may connect to the webbings along the anterior aspect of the limb, which may help hold the various webbings in orientation with respect to each other and/or the anterior struts. This may be made of another textile material, mesh, webbing, foam, Velcro, plastic, laminate, or other materials, which may generally help to connect the various webbings together proximal to distal. This insert may as well be partially connected to the struts on its proximal end to help encourage the anterior aspect of the webbing to provide more posting support distally versus proximally. In addition, the insert may include suspension mechanisms incorporated within to help hold the prosthetic in place. For instance, this insert may include a unidirectional friction element, which may help suspend the prosthetic on the limb, through the insert's inherence compliance and conformity to the limb shape. Since the insert, along with the attached webbing, may be so conforming to the limb shape it may provide greater capture of the intimate contouring of the limb, and therefore, allow such friction, or other suspension elements, to hold onto the limb/liner with greater effectiveness.

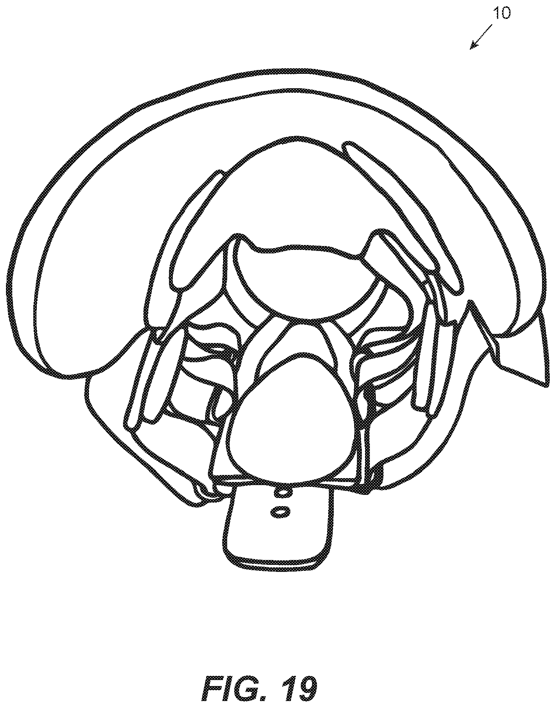

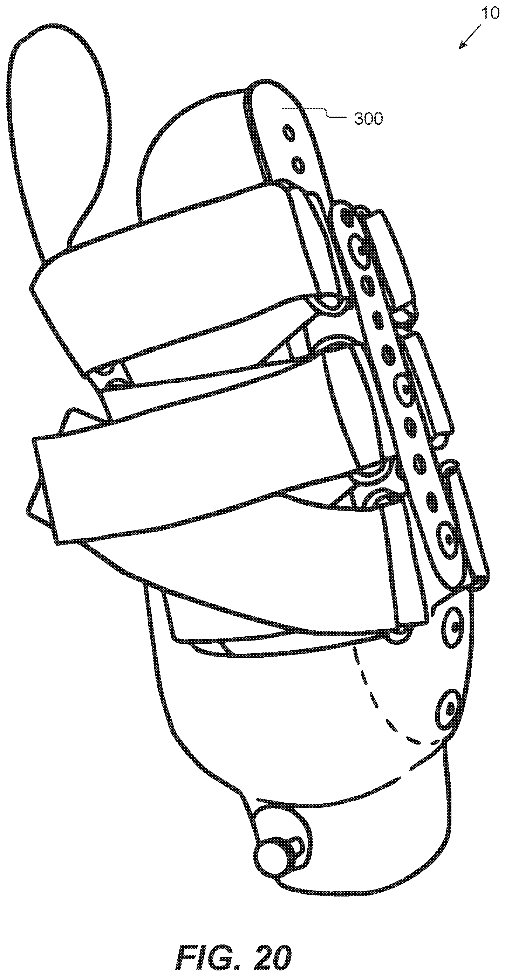

Through integrating the floating webbing within the socket to support the limb and post it away from the struts, the limb may predominantly be supported by compliant dynamic materials versus rigid structure more so than any other socket available. Likewise, the angles of the various layers of webbing as they route though the directional loops may encourage more tissue containment, as the overlapping spans of webbing at different angles may provide greater coverage over the limb and may prevent limb tissue from protruding through - as illustrated in Figure 19, as viewed from looking into the socket from above; and Figure 20, as viewed from looking at the side of the socket. In each illustration, the direction and angle of each layer of webbing is such that the underlying layer crosses at an angle compared to the overlying layer such that it may help cover the span in between the outer webbing layers. This may provide additional tissue containment.

On the posterior side of the socket, a flexible member 300 may extend proximally up from the posterior strut, and extend past the patellar tendon level in height (which is typically associated with the level of posterior brim of a below knee socket). The flexible member may provide additional comfort during knee flexion, by allowing the posterior brim of the socket to have a larger radius in shape versus a trimmed edge of a socket material cutting into the fold in the knee.

It is contemplated that various forms of adjustor mechanisms may be used to help tighten the webbings around the limb, including hand-tightening, or mechanical or electro-mechanical tightening. In a preferred embodiment, the unique modularity of such technology may allow for any form of tightening to occur, based on end-users' needs. However, in order to provide a more elegant user experience, managing the ends of the webbing within an adjustor mechanism may be advantageous, versus hand-tightening by pulling on a tail end of the webbing.

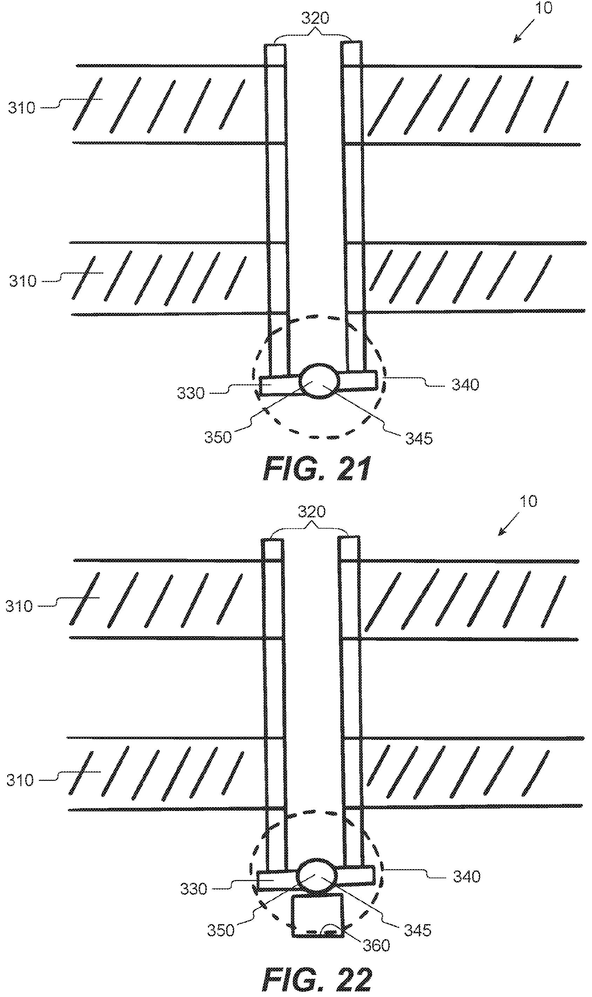

Figures 21 and 22 illustrate one embodiment of an adjuster mechanism, which may help manage the webbing. In such an example webbing sections 310 may be connected to at least one shaft 320. The other end of such webbing may loop around the limb similar to any of the Figures 13 through 20 examples. Connected to the shafts 320 may be a gear mechanism 330, which may enable the user to tighten the webbing around the shafts 320 with a dial or other such ergonomic method 340, and which may use a worm gear 350 or other such gearing methods to transfer the rotation of the shafts 320 perpendicular to match the dial 340.

In a preferred embodiment, such mechanism may be low profile, durable, simple to use, ergonomic, non-backdriveable, among other characteristics. While various methods may be used to accomplish such characteristics, the illustration and explanation should not be considered limiting but is used for example and ease of understanding.

Through using a non-backdrivable gear mechanism 345, such as but not limited to a worm gear 350, a simple positive-locking positioning of the webbing may be achieved without the need of other locking mechanisms. An ergonomic dial may be used in-line with the worm gear for ease of adjustability. And shafts may be integrated with the gear mechanisms to roll up the webbing. Such an assembly may be mounted along the length of one of the struts.

As is illustrated in Figure 22, the further integration of additional gears and/or motor, sensor(s), and battery, altogether motor assembly 360 may provide a self-adjusting means of tightening the socket. **In** such an example, motor assembly 360 may be controlled through a microprocessor or the like along with at least one sensor to help control the device including but not limited to time sensor, motor position sensor, motor torque sensor, accelerometer or the like, force sensor, or other such sensors. Upon the tightening of the webbing through the motor and controller, the socket fit may be tightened, and vice versa with loosening the socket.

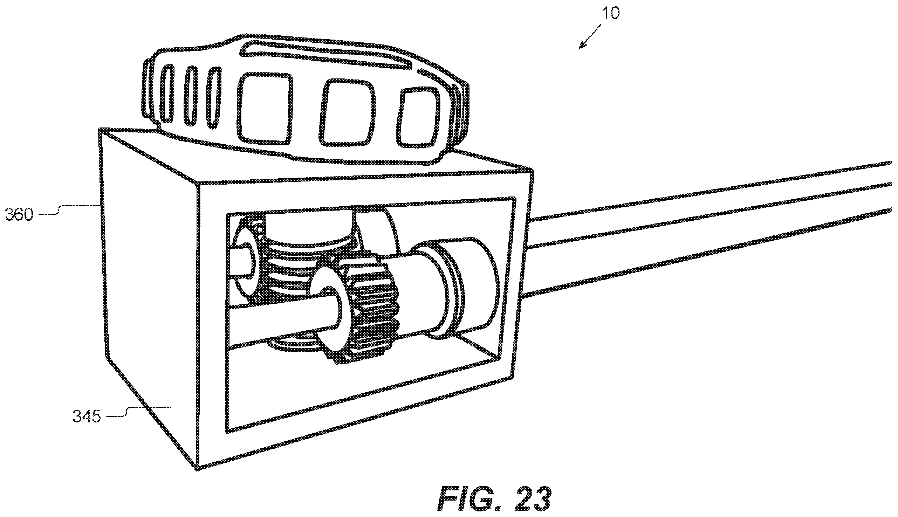

Figure 23 illustrates a perspective view as represented in Figures 21 and 22, of what such a gear mechanism 345 may resemble. **In** such an example, dial may be connected to worm gear, which may interface with gears connected to shafts, upon which such webbing may role up on as the dial is turned. Such a unit may be mounted at various locations on a socket, although in a preferred embodiment, upon a strut, such as the posterior floating or connected strut of a below knee socket for example.

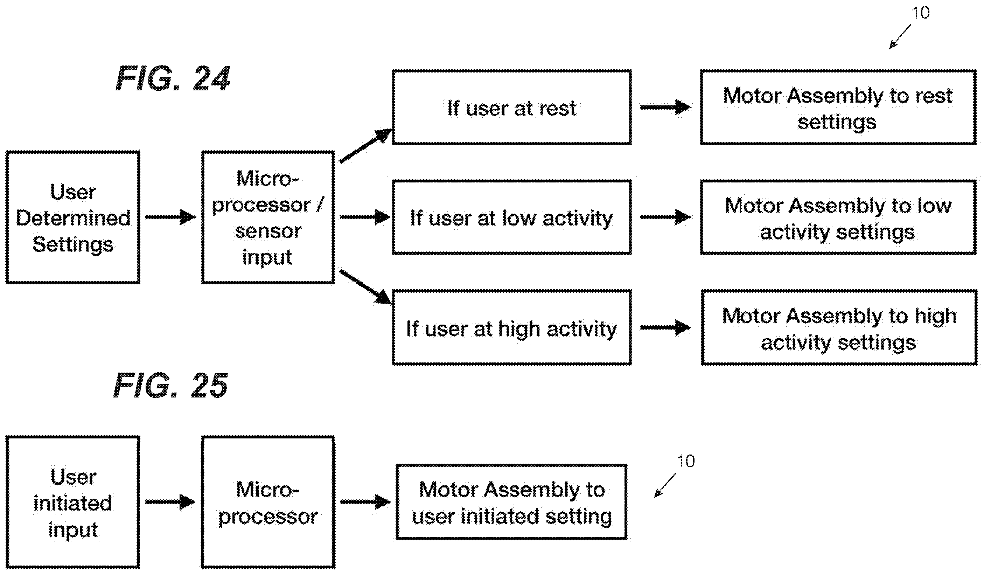

As illustrated in Figures 24 and 25, user initiated changes to the motor position may be used, or autonomous control mechanisms may be used. User initiated means may include a user inputting directions to smart phone or push button to direct the motor assembly movement or position. Autonomous control mechanisms may include sensor input to direct the motor assembly movement or position.

In a preferred embodiment, the user may program set for autonomous control of the device a maximum socket tightness and a minimum socket tightness, may set activity related socket tightnesses, or other such means of controlling the socket's webbing position. When a user dons the socket, autonomous or a user initiated input may begin the tightening of the socket to a pre-set tightness positioning or pre-set user preferred force positioning of the webbing, and vice versa for doffing the socket. Sensor input may help determine the user's activities such as resting, walking, running, or others, and may be used to adjust the socket tightness or positioning as it relates to the user's activities.

For example, when the user is in a resting position the socket may loosen slightly, whereas when the user is walking or running the socket tightness may be greater. The socket may as well provide for cyclical tightening and loosening throughout the day to help promote blood flow to the limb or other reasons such as re- assessing torque values on a motor to correspond to proper tightening of the socket to the existing limb shape. As the limb may change in volume throughout the day, the webbing may as well tighten to accommodate for a reduction in limb volume for instance. It may also have a self-learning algorithm, whereas the user may provide input through a push button, for example, and a self-learning algorithm may correlate their activity levels or socket tightness with the user initiated socket changes, and allow the socket system to self-learn the user's preferences of socket fit based on user activities, and then may perform such socket adjustments upon learning the user's preferences via their input. This may also correlate via number of steps, for instance the user may typically input user preferred tightening of the socket after about 3000 steps on average, and so the socket may automatically tighten a comparable amount after 3000 steps, which may be driven by sensor input and autonomous control. Likewise, when sensor input such as an accelerometer or the like may indicate the user begins to run, the socket may automatically tighten slightly whereas when the user is at rest it may loosen slightly.

Since various sensors may be integrated within the system, data collection may be captured including, but not limited to, number of steps, timing of steps, cadence including varied cadence, user specific activities, activity level of the user, times or use or non-use, and socket forces, amongst others. Likewise, the autonomous control of the socket fit may provide automatic socket adjustments to fit, self-learning adjusting, accommodation for volume change, user preference/learning algorithm, and others, which may be displayed to the user.

Fitting a socket may as well include the process of forming modular components onto the limb shape, such as but not limited to formable struts or non-formable struts, which may be fabricated to shape, taking an impression of the distal end of the limb to generate a connection point between such struts and the distal componentry, taking an impression of the proximal end the limb such as around the 'ears' of a below knee socket as needed, and fitting such impression-created forms onto the struts for assembly of the structural elements of a socket, and finally to assemble the other complaint or dynamic modular components of the socket to create a socket in its entirety. In such an example, the impressions may include materials such as cutter-cast, fiberglass, other laminates, thermoplastics, or similar, which may even be formed to the limb shape in real-time, and may be used directly for the fitting, versus using an impression to then later fabricate the same shape from other materials. By using a real-time usable material, only minor trim line adjustments may be needed to these structural elements, and the socket system can be fit in real-time along with the other dynamic socket components. Since the dynamic socket materials may as well be modular in nature, fitting a socket in its entirety using this method enables for the impression, fabrication, and fitting to all take place in a single appointment without the need of additional fabrication methods that conventional sockets require.

Using truly conforming compliant or dynamic socket materials as is described, along with the self-adjustable elements of the socket shape enables the limb muscles to no longer hit a rigid wall, as is experienced in using conventional prosthetics, and instead promoted limb muscle use and growth, increased circulation, and overall better limb health.

Additionally, with so many elements of the socket being conforming, compliant, and dynamic to better contour to the limb shape, integrating a dynamic suspension lock that moves with the limb versus mounted to a rigid socket may additionally help provide added comfort. Conventional socket lock mechanisms are commonly mounted to the rigid frame structure to provide a rigid connection point. In the disclosed socket design however, the distal receptacle, which dynamically conforms to the limb shape, may include a lock mechanism mounted to it, which may enable the user's limb to connect thereto and provide a more dynamic suspension mechanism than in conventional sockets, as the lock mechanism may move with the limb movement within the socket.

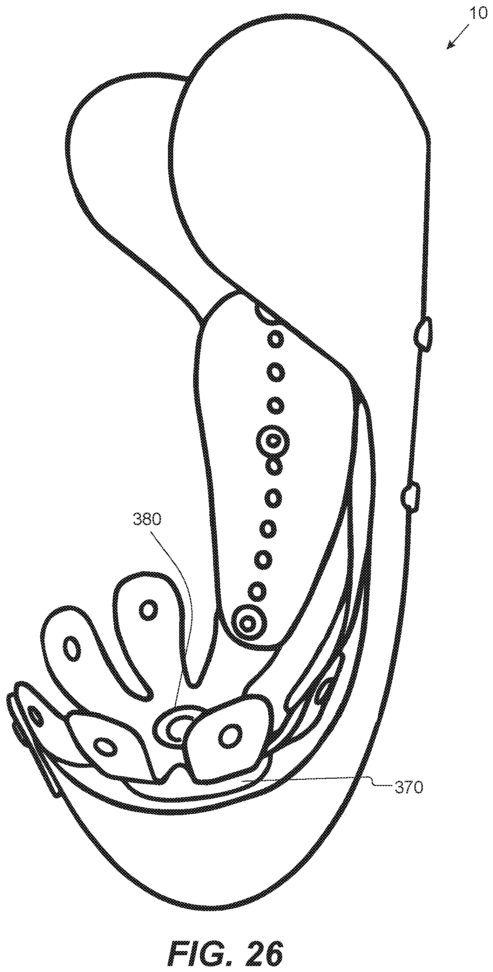

This provides a more conforming method of suspension at the sensitive distal end of the limb. In such an example, and as illustrated in Figure 26, a lock mechanism 370 may be generally connected to the functional equivalent of a receptacle 380 as shown, and allow the limb/liner to engage with the lock mechanism 370 in a certain location, such as illustrated in position 380. The lock mechanism 370 may be a pin system, a lanyard system, a vacuum or suction suspension system, or other such suspension mechanisms known in the field. The receptacle 380 may provide conformity and dynamic nature with the limb, but may also have enough form-factor to hold a relative position within the socket. Too much flexibility in the part may limit true suspension ability, while too much rigidity may fail to accommodate for micro-movements of the limb within the socket and put excessive pressure on the sensitive distal end of the limb. Providing a suspension mechanism or system that slightly moves with the limb may soften the connection point between the sensitive limb end and the socket, providing more comfort for the user.

Invention 10 may comprise a transfemoral prosthetic interface having a circumference for a residual limb having a circumference and a distal end comprising a socket having a first side and a second side; a floating strut between said first side of said socket and said second side wherein said floating strut does not touch said first side or said second side of said socket; a lacing system connected to said first side and said second said of said socket and adapted to hold said floating strut between said first side and said second side of said socket; and wherein said socket, said floating strut, and said lacing system form a circumference are adapted to support said residual limb around said circumference of said residual limb without putting pressure on said distal end of said limb.

Changes may be made in the combinations, operations, and arrangements of the various parts and elements described herein without departing from the scope of the invention. Furthermore, names, titles, headings and general division of the aforementioned are provided for convenience and should, therefore, not be considered limiting.

## Claims

1. A transfemoral prosthetic interface (10) having a circumference for a residual limb having a circumference and a distal end comprising:
a socket (25) having a first side (20) and a second side (30);
a floating strut (40) between said first side of said socket (25) and said second side
wherein said floating strut does not touch said first side (20) or said second side (30) of said socket;
a lacing system (70) connected to said first side (20) and said second side (30) of said socket, said lacing system adapted to hold said floating strut (40) between said first side and said second side of said socket (25); and
**characterised in that** the lacing system (70) comprises a ratchet and a ladder strap (80) and
a plurality of self-levelling laces, and wherein said socket (25), said floating strut (40), and said lacing system (70) form a circumference and are adapted to support said residual limb around said circumference of said residual limb without putting pressure on said distal end of said limb by suspending the limb within the socket (25).

2. A transfemoral prosthetic interface according to claim 1, further comprising a webbing system.

## Patentansprüche

1. Oberschenkelprothesenanbindung (10), die einen Umfang für einen Stumpf aufweist, der einen Umfang und ein distales Ende aufweist, die Folgendes umfasst:
einen Schaft (25), der eine erste Seite (20) und eine zweite Seite (30) aufweist;
eine schwebende Strebe (40) zwischen der ersten Seite des Schafts (25) und der zweiten Seite,
wobei die schwebende Strebe die erste Seite (20) oder die zweite Seite (30) des Schafts nicht berührt;
ein Schnürsystem (70), das mit der ersten Seite (20) und der zweiten Seite (30) des Schafts verbunden ist, wobei das Schnürsystem geeignet ist, um die schwebende Strebe (40) zwischen der ersten Seite und der zweiten Seite des Schafts (25) zu halten; und
**dadurch gekennzeichnet, dass** das Schnürsystem (70) eine Ratsche und einen Leitergurt (80) und eine Vielzahl von selbstregulierenden Schnürriemen umfasst, und wobei der Schaft (25), die schwebende Strebe (40) und das Schnürsystem (70) einen Umfang bilden und geeignet sind, um den Stumpf um den Umfang des Stumpfes herum zu tragen, ohne Druck auf das distale Ende des Glieds durch Aufhängen des Glieds innerhalb des Schafts (25) auszuüben.

2. Oberschenkelprothesenanbindung nach Anspruch 1, die weiter ein Gurtbandsystem umfasst.

## Revendications

1. Interface prothétique transfémorale (10) ayant une circonférence pour un membre résiduel ayant une circonférence et une extrémité distale comprenant :
une douille (25) ayant un premier côté (20) et un deuxième côté (30) ;
une entretoise flottante (40) entre ledit premier côté de ladite douille (25) et ledit deuxième côté
dans laquelle ladite entretoise flottante ne touche pas ledit premier côté (20) ou ledit deuxième côté (30) de ladite douille ;
un système de laçage (70) connecté audit premier côté (20) et audit deuxième côté (30) de ladite douille, ledit système de laçage conçu pour maintenir ladite entretoise flottante (40) entre ledit premier côté et ledit deuxième côté de ladite douille (25) ; et
**caractérisée en ce que** le système de laçage (70) comprend un cliquet et une sangle en échelle (80), et une pluralité de lacets à nivellement automatique, et dans laquelle ladite douille (25), ladite entretoise flottante (40), et ledit système de laçage (70) forment une circonférence et sont conçus pour supporter ledit membre résiduel autour de ladite circonférence dudit membre résiduel sans exercer de pression sur ladite extrémité distale dudit membre en suspendant le membre au sein de la douille (25).

2. Interface prothétique transfémorale selon la revendication 1, comprenant en outre un système de sangle.
